# EUROPÄISCHE PATENTSCHRIFT

(11) **EP 2 588 035 B1**
(45) Veröffentlichungstag und Bekanntmachung des Hinweises auf die Patenterteilung: **07.09.2016**
(21) Anmeldenummer: 11738693.8
(22) Anmeldetag: 25.07.2011
(51) Int. Cl.: A61F 2/28, A61F 2/30, A61F 2/44, A61F 2/46

(54) **IMPLANTAT FÜR DIE WIRBELSÄULE UND BETÄTIGUNGSINSTRUMENT**
IMPLANT FOR THE SPINAL COLUMN AND ACTUATING INSTRUMENT
IMPLANT POUR LA COLONNE VERTÉBRALE ET INSTRUMENT D'ACTIONNEMENT

(30) Priorität: 11.10.2010 DE 102010047901
(43) Veröffentlichungstag der Anmeldung: 08.05.2013
(73) Patentinhaber: Böhm, Heinrich, Dr., 99425 Weimar (DE)
(72) Erfinder: BURGER, Andreas, 78532 Tuttlingen (DE); WENZLER, Klaus, 78665 Frittlingen (DE)
(74) Vertreter: Mammel und Maser
(86) Internationale Anmeldenummer: PCT/EP2011/062719
(87) Internationale Veröffentlichungsnummer: WO 2012/048920

(56) Entgegenhaltungen:
- EP-A1- 1 878 408
- WO-A2-03/032812
- DE-A1-102008 010 607
- FR-A1- 2 850 563
- US-A1- 2005 080 422
- US-A1- 2007 255 409

## Beschreibung

Die vorliegende Erfindung betrifft eine Implantatvorrichtung umfassend ein höhenverstellbares Implantat für die Wirbelsäule zur Rekonstruktion von Wirbelsäulendefekten und ein Betätigungsinstrument. Insbesondere dient das Implantat dazu, Teile der Wirbelkörper und angrenzende Bandscheiben zu ersetzen.

Die bislang bekannten Corpektomie-Implantate basieren auf dem Wagenheberprinzip und ermöglichen somit eine Höhenverstellbarkeit. Nachteilig an diesen Implantaten ist jedoch, dass eine knöcherne Durchbauung der Implantate kaum möglich ist, da diese hülsenförmig aufgebaut sind und somit wenig bis keinen Kontakt zum gesunden Knochen erlauben und auch wenig biologischen Reiz zur Knochenneubildung liefern. Auch nach dem Verheilen der Wunde lastet in diesen Fällen die gesamte Last auf dem Implantat, was zu Ermüdungsbrüchen der Deckplatten der Verankerungswirbel mit Einsinken des Implantats und schmerzhaften Fehlstellungen bis hin zur Gefahr der Querschnittslähmung durch Kompression des Rückenmarks führen kann.

Solche hülsenförmig aufgebauten, höhenverstellbaren Implantate sind aus der DE 196 04 246 B4 und der DE 202 07 853 U1 bekannt.

Ein weiterer Nachteil dieser Implantate ist, dass die Veränderbarkeit des Implantats in der Höhe ausschließlich linear erfolgen kann, d.h. das Implantat erlaubt nicht die Option, beispielsweise im Halsbereich der natürlichen leichten Krümmung - der Lordose - zu folgen.

Ein weiterer Nachteil der bislang bekannten Implantate ist, dass sie bauartbedingt nicht den Rückenmarkskanal gegen eine erneute Einengung sichern können, beispielsweise durch unbeabsichtigte Verlagerung von Knochenspänen am Implantat vorbei oder ersatzweise eingebrachtem Knochenzement.

Nachteilig an diesen Implantaten nach dem Wagenheberprinzip ist weiterhin, dass sie einsinken, insbesondere, wenn keine zusätzliche knöcherne Versteifung entsteht.

Aus der US 6,562,074 B2 ist ein rechteckförmiges höhenverstellbares Implantat bekannt, das aus einem Außen- und einem Innenkorb und einem Verstärkungselement besteht. Infolge des Verstärkungselements lässt dieses Implantat keine Durchknöcherung zu. Außen- und Innenkorb sind bei dieser Vorrichtung so zueinander ausgerichtet, dass jeweils die Böden der U-förmigen Außen- und Innenkörbe die Wirbelstützflächen bilden.

Aus der US 2005/0080422 A1 ist ein höhenverstellbares Implantat mit einem U-förmigen Außen- und einem U-förmigen Innenkorb bekannt, bei dem der innere Korb von dem äußeren Korb umgriffen und teleskopartig geführt wird. Die Böden des Innen- und Außenkorbs liegen einander gegenüber und bilden die Wirbelstützflächen. Eine knöcherne Durchbauung ist bei diesen Implantaten nur eingeschränkt möglich.

Weiterhin sind Corpektomie-Implantate bekannt, die die Form eines U-förmigen Cages aufweisen. Diese Implantate sind mit Knochenmaterial befüllbar, so dass eine Verknöcherung des Implantats erreicht werden kann. Eine Höhenverstellbarkeit dieser Implantate ist als solche nicht vorgesehen. Die Anpassung der Implantate an die erforderliche Höhe erfolgt bei diesen Implantaten derart, dass diese während der Operation auf die gewünschte Länge gekürzt und die Außenkanten dann ein wenig abgerundet werden. Nachteil an diesen Implantaten ist, dass diese in den Wirbelkörper einsinken, was zu Fehlstellungen und erneuten Einengungen von Nervenstrukturen/Rückenmark oder Beeinträchtigungen/Versagen der Nachbarbandscheiben führen kann.

Ein weiterer Nachteil dieser Implantate besteht darin, dass die Längenbestimmung häufig schwierig ist und die Kürzung nicht auf die passende Länge erfolgt, so dass das Implantat wieder entnommen und ein weiteres Implantat implantiert werden muss.

Weiterhin ist aus der EP 1 267 755 B1 ein höhenverstellbares Wirbelkörperimplantat bekannt, das aus einem ersten im Wesentlichen U- oder C-förmigen Korb sowie am Korb ausgebildeten Wirbelstützflächen besteht. Der erste Korb ist ein innerer Korb, der von einem zweiten äußeren U-oder C-förmigen Korb umgriffen und teleskopartig geführt wird, wobei beide U-förmigen Körbe gleich ausgerichtet sind und das aus beiden Körben gebildete Implantat ebenfalls U=förmig ist. Die Schenkel des inneren und des äußeren Korbes sind so ausgerichtet, dass sich eine durchgehende Öffnung ergibt, die den Böden der Körbe gegenüberliegt. An den Stirnseiten der Körbe sind Wirbelstützflächen vorgesehen. Der innere Korb weist weiterhin in dem die Schenkel verbindenden Bereich ein sich in Längsrichtung erstreckendes Langloch mit einer einseitigen Verzahnung auf. Die Verschiebung der beiden Körbe gegeneinander erfolgt mittels eines Betätigungsinstruments mit einer Außenverzahnung.

Ist die gewünschte Höhe erreicht, so werden die Körbe in der jeweiligen Position durch drei Schrauben gesichert.

Das Implantat ist höhenverstellbar und kann mit Knochenmaterial durch die durchgehende Öffnung befüllt werden und somit knöchern einheilen. Zudem sinkt das Implantat nur geringfügig in den Wirbelkörper ein.

Die Fixierung der beiden Körbe erfolgt mit Schrauben in Richtung Rückenmark. Durch die Wandverstärkungen des äußeren Korbs im Bereich der Fixationsschrauben weist zudem die dem Rückenmark/der Dura zugewandte Seite eine Vorwölbung auf, die entweder eine örtliche Dura-Irritation auslösen kann oder eine Positionierung in mehr Abstand zum Rückenmark als optimal erzwingt.

Weiterhin ist an diesem Implantat nachteilig, dass es bei dem - am häufigsten vorkommenden - Einsatz von nur einem Wirbelkörper z.B. im Halsbereich bauartbedingt einen zu geringen Aufspreizweg aufweist und sich daher nicht sicher verankern lässt.

Auch für die Anwendung im oberen Brustwirbelsäulenbereich erweist sich das Missverhältnis zwischen Aufspreizbarkeit und erforderlichem Aufspreizweg bei diesem Implantat oft als Ausschlusskriterium für die Anwendung in transthorakaler (ventraler) Technik.

Für die operative Option eines Einbringens von dorso-lateral (von hinten seitlich am Rückenmark vorbei) gibt es neben der zu geringen Aufspreizbarkeit auch die Schwierigkeit, dass der Aufspreizmechanismus "um die Ecke" bei 90 Grad verdrehtem Implantat nicht zur Anwendung kommen kann.

Ein weiterer Nachteil des bekannten Implantats ist die hohe Strahlungsbelastung für den Patienten während des Einbringens.

Als cervicales Implantat ist dieses Implantat darüber hinaus wegen des hohen Platzbedarfs des Verschiebe- und Fixierungsmechanismus und der daraus resultierenden großen Baugröße nicht geeignet.

Die DE 10 2008 010 607 lehrt ein höhenverstellbares Wirbelkörperimplantat mit einem U-förmigen Außenkorb und einem in dem Außenkorb geführten U-förmigen Innenkorb in lordoser Form. Das Implantat ist U-förmig. Die Fixierung erfolgt durch Verrastung der an den beiden Körben angeordneten korrespondierenden Verzahnungen. Vor der Verrastung weist die an dem Innenkorb vorgesehene Verzahnung leicht in Richtung des Korbinneren, so dass sich der Innenkorb gegenüber dem Außenkorb verschieben lässt. Ist die gewünschte Position erreicht, werden die an dem Innenkorb vorgesehenen Verzahnungen leicht nach außen gegen die Verzahnungen des Außenkorbs mittels eines Sperrelements gepresst und somit ein selbsthaltender Sitz erreicht.

Bei diesem Implantat ist die Aufspreizrichtung nicht auf die Einbringrichtung beschränkt. Zudem werden Irritationen der Dura vermieden und infolge der lordosen Form sind keine Zusatzimplantate erforderlich. Dieses Implantat eignet sich auch für den Halsbereich.

Nachteilig ist an diesem Implantat jedoch, dass das Aufspreizen der beiden Körbe, d.h. die Relativbewegung der beiden Körbe zueinander und ein Verstellen der beiden Körbe entlang der der lordotischen Form entsprechenden geringen Krümmungslinie Schwierigkeiten mit sich bringt. Zudem kann nach einer einmal erfolgten Verrastung des Innenkorbes mit dem Außenkorb eine überspreizte Position sehr schwer wieder rückgängig und der Innenkorb gegenüber dem Außenkorb wieder in "Verschlebestellung" gebracht werden.

Die Aufgabe der vorliegenden Erfindung besteht darin, höhenverstellbare, mit Knochen oder Knochenersatzmaterial befüllbare Implantate, die als Corpektomie-Korb einsetzbar sind und deren Aufspreizrichtung nicht von der Einbringrichtung abhängt, bereitzustellen, die einfach bedienbar und bei denen auch eine Überspreizung nachträglich wieder auf einfache

Weise rückgängig gemacht werden kann und ein Betätigungsinstrument hierfür anzugeben.

Diese Aufgabe wird durch die Merkmale des Anspruchs 1 gelöst.

Erfindungsgemäß umfasst das Betätigungselement einen Fixierungsstab und einen Spreizmechanismus zur Betätigung von Schiebeelementen, und am Innenkorb des Implantats und am Fixierungsstab sind korrespondierende Elemente eines lösbaren Verbindungselements vorgesehen, so dass der Fixierungsstab mit dem Innenkorb starr verbindbar ist. Durch Betätigung des Spreizmechanismus ist der Außenkorb gegenüber dem Innenkorb durch die Schiebeelemente in Längsrichtung (L-Richtung) verschiebbar, wobei der Angriff der Schiebeelemente an den Außenkorb an den Schenkeln erfolgt. Vorzugsweise greifen die Schiebeelemente an der gegenüber der Stirnseite des Außenkorbs befindlichen Seite der Schenkel an, so dass nur eine geringe Spreizbewegung erforderlich ist und die Sicht und das Operieren nicht durch sperrige, die Sicht behindernde Instrumente behindert wird.

Als innenkorbseitiges Verbindungselement ist vorzugsweise am Boden des Innenkorbs eine Gewindebohrung vorgesehen, die der Aufnahme eines korrespondierenden Außengewindes an dem Fixierungsstab des Betätigungsinstruments, das heißt dem korrespondierenden Verbindungselement, dient. Wird der Fixierungsstab mit seinem Außengewinde in die Bohrung im Boden des Innenkorbs eingeschraubt, so wird eine starre Verbindung zwischen Fixierungsstab und innerem Korb erreicht.

Nachdem jedoch das Betätigungsinstrument zusätzlich auch einen Spreizmechanismus mit Schiebeelementen aufweist, bewirkt die Betätigung des Spreizmechanismus eine Verschiebung der Schiebeelemente, insbesondere Hebelenden, in Längsrichtung (L-Richtung) gegenüber dem Fixierungsstab mit Innenkorb und damit eine Verschiebung des Außenkorbs gegenüber dem Innenkorb.

Durch eine Entlastung des Spreizmechanismus können die Schiebeelemente wieder in L-Richtung verschoben und damit eine Überspreizung zwischen Außen- und Innenkorb wieder aufgehoben werden.

Um eine der Lordoseform folgende Spreizbewegung der beiden Körbe gegeneinander zu erreichen, weisen die Außenseiten der Schenkel des Innenkorbs vorzugsweise eine sich in Längsrichtung erstreckende Stützfläche auf, an der sich die Schiebeelemente, die beispielsweise Hebelenden sein können, während der Betätigung des Spreizmechanismus abstützen. Durch dieses Merkmal wird eine gute Führung erreicht.

Eine weitere Verbesserung der Führung der beiden Körbe bei der Spreizbewegung kann dadurch realisiert werden, dass auf der der Stirnseite des Außenkorbs gegenüberliegenden Seite der Schenkel eine Stufe vorgesehen ist, die der Abstützung der Schiebeelemente des Betätigungsinstruments dienen.

Um die Positionierung von weiteren Instrumenten wie beispielsweise Inbusschlüsseln oder Gegenhaltern in der seitlichen Öffnung des Implantats zu erleichtern und ein Verrutschen der Bedieninstrumente zu verhindern, weist der Außenkorb des Implantats vorzugsweise einen die offene Seite überbrückenden Überbrückungssteg auf, der mit den Schenkeln des Außenkorbs fest verbunden ist und der Ausnehmungen zur Anlage von Instrumenten umfasst.

Der Überbrückungssteg deckt nur einen kleinen Bereich der Öffnung ab, um die Sicht während der Implantation so wenig wie möglich zu behindern.

Der Einsatz von Zusatzimplantaten kann entfallen, da das erfindungsgemäße Implantat eine der lordotischen Form entsprechende geringe Krümmungslinie aufweist. Um die Verschiebung der beiden Körbe gegeneinander entlang der lordotischen Krümmungslinie zu erreichen und ein Verkanten zu verhindern, sind an den Körben, vorzugsweise an deren Schenkeln, Elemente vorgesehen, die zueinander korrespondieren und die der Führung der Spreizbewegung in einer der lordotischen Form folgenden Bewegung dienen. Diese Führungselemente können als korn respondierende Stützflächen im Außen- und Innenkorb, aber auch durch Öffnungen, insbesondere der Lordoseform folgende Langlöcher mit in diesen Öffnungen geführten Vorsprüngen, ausgebildet sein. Auch andere korrespondierende Führungsmittel sind möglich, solange diese eine Führung entlang der Lordoseform ermöglichen. Die in den Öffnungen geführten Vorsprünge dienen - wie der Überbrückungssteg - auch dazu, ein Herausfallen des Innenkorbs aus dem Außenkorb senkrecht der Längsrichtung zu verhindern.

Ist die gewünschte Endposition in Verschieberichtung erreicht, so wird der Innenkorb gegenüber dem Außenkorb fixiert. Die Fixierung kann verschiedenartig erfolgen, vorzugsweise durch Verschraubung. In einer besonders bevorzugten Ausführungsform weist der Boden des Innenkorbs ein Langloch und der Außenkorb im Boden verschiedene Öffnungen auf, die der Verschraubung dienen.

Um Ermüdungsbrüche der Deckplatten der Verankerungswirbel zu vermeiden, weist das erfindungsgemäße Implantat vorzugsweise mehrere Öffnungen in den Schenkeln, im Boden und den Wirbelstützflächen auf, die der besseren Durchknöcherung dienen.

Das Betätigungsinstrument umfasst einen Fixierungsstab und einen Spreizmechanismus, wobei in einer bevorzugten Ausführungsform am Ende des Fixierungsstabs das Außengewinde, das zum Einschrauben in die Bohrungen am Boden des Innenkorbs dient, vorgesehen ist.

Um eine gute Kraftübertragung zu erreichen, Verkantungen zu verhindern und die Größe des Betätigungsinstruments so gering wie möglich zu halten, weist das Betätigungsinstrument die Schiebeelemente und den Spreizmechanismus vorzugsweise am Ende des Fixierungsstabs auf.

Der Spreizmechanismus sollte gegenüber dem Fixierungsstab verschiebbar sein, um die Betätigung des Spreizmechanismus und damit eine Schwenkbewegung der Betätigungselemente bzw. die Verschiebebewegung der Schiebeelemente gegenüber dem Fixierungsstab zu ermöglichen.

In einer bevorzugten Variante umfasst der Spreizmechanismus ein Kraftübertragungselement mit einer schrägen Druckfläche an der Unterseite. Die Hebelelemente, die die Schiebeelemente umfassen, sind schwenkbar um eine Achse gelagert. Die Schwenkachse befindet sich nahe des Stabendes, so dass die Schwenkbewegung recht klein ist, zumal die Enden der Schiebeelemente oberhalb des Endes des Fixierungsstabs enden. Erstaunlicherweise ermöglicht diese kleine Schwenkbewegung des Schiebeelements die Verschiebung der Körbe. Im Ruhezustand liegt das obere Ende der Hebelelemente an der Druckfläche an, so dass bei Krafteinleitung durch die schräge Druckfläche des Kraftübertragungselements das untere Ende des Hebelelements, nämlich das Verschiebeelement, aus der Ruhelage heraus geschwenkt werden kann.

Das Kraftübertragungselement ist von einem U-förmigen Gehäuse umgeben, das Gehäuse weist ein sich längs der Achse des Fixierungsstabs erstreckendes Langloch auf, in dem ein das Langloch und Kraftübertragungselement durchquerender Fixierungsstab geführt ist.

Das untere Ende des Gehäuses wird von einer Achse durchquert, an der die Hebelelemente schwenkbar befestigt sind.

Die Betätigung des Spreizmechanismus erfolgt mit einer entlang des Fixierungsstabs nach unten wirkenden Kraft. Vorzugsweise weist der Fixierungsstab eine Gewindespindel auf, auf deren Außengewinde eine Betätigungshülse aufgeschraubt werden kann. Durch die Schraubbewegung drückt die Unterkante der Betätigungshülse das Kraftübertragungselement in Richtung Stabende und bestätigt dadurch den Spreizmechanismus.

Durch die Gewindespindel kann eine genaue Dosierung der auf das Kraftübertragungselement wirkenden Kraft erzielt werden.

Der erfindungsgemäße Spreizmechanismus sieht weiterhin vor, dass der Fixierungsstab eine Positionierscheibe aufweist, die nahe des Stabendes verdrehbar, jedoch unverschiebbar gelagert ist, und dass der Verschiebeweg des Spreizmechanismus entlang dem Fixierungsstab durch die Positionierscheibe und die Gewindespindel begrenzt ist und der Spreizmechanismus zusammen mit der Positionierscheibe gegenüber dem Fixierungsstab drehbar ist, der Spreizmechanismus jedoch gegenüber der Positionierscheibe nicht drehbar ist.

Vorzugsweise weist die erfindungsgemäße Implantatvorrichtung zusätzlich einen Gegenhalter zur Aufnahme des auf das Implantat wirkenden Drehmoments auf, der in der Ausnehmung in dem Überbrückungssteg positioniert werden kann.

Ein besonderes Merkmal der Erfindung ist weiterhin, dass das Implantat zuerst optimal platziert werden kann und anschließend die nicht zur Abstützung erforderlichen Flächen der Ankerwirbel angefrischt werden können. Dies vermeidet die Schwächung des Implantatlagers durch Anfrischen der Endplatten vor der Implantation, wie das bei den durch den Stand der Technik bekannten Implantaten bauartbedingt erforderlich ist. Bei optimaler Anfrischung der Nachbarwirbel kommt es zu einer raschen Inkorporation des Implantats und Verringerung der Komplikationsmöglichkeiten (Einsinken, verzögertes oder fehlendes Einwachsen).

Ein weiterer Vorteil der vorliegenden Erfindung besteht darin, dass der Aufspreizmechanismus auch "um die Ecke" bei einem 90 Grad verdrehten Implantat zur Anwendung kommen kann, da die Öffnungen zum Eingriff der Instrumente seitlich angeordnet sind und bei dem erfindungsgemäßen Implantat keine bodenseitige Fixierung erfolgen muss.

Die vorliegende Erfindung zeichnet sich auch dadurch aus, dass das Implantat mit einem Einbringinstrument eingebracht wird und das Einbringinstrument bei der Röntgenkontrolle nicht störend ist.

Die Erfindung wird nachfolgend anhand von Ausführungsbeispielen näher beschreiben.

Es zeigt:
- Figur 1:: eine perspektivische Ansicht des Implantats mit einem im Außenkorb geführten Innenkorb mit Schraube,
- Figur 2:: eine perspektivische Ansicht des Innenkorbs,
- Figur 3:: eine Seitenansicht des Innenkörbes,
- Figur 4:: eine Draufsicht auf den Innenkorb von unten,
- Figur 5:: eine Draufsicht auf die Stirnseite des Innenkorbs,
- Figur 6:: eine perspektivische Ansicht des Außenkorbs,
- Figur 7:: eine perspektivische Ansicht des Außenkorbs von schräg unten,
- Figur 8:: eine Draufsicht auf den Außenkorb von oben,
- Figur 9:: eine Draufsicht auf die Längsseite der Schenkel des Außenkorbs,
- Figur 10:: einen Schnitt durch Figur 1 entlang Linie A-A mit Schrauben,
- Figur 11:: eine perspektivische Ansicht auf die Stirnseite des Innenkorbs des Implantats,

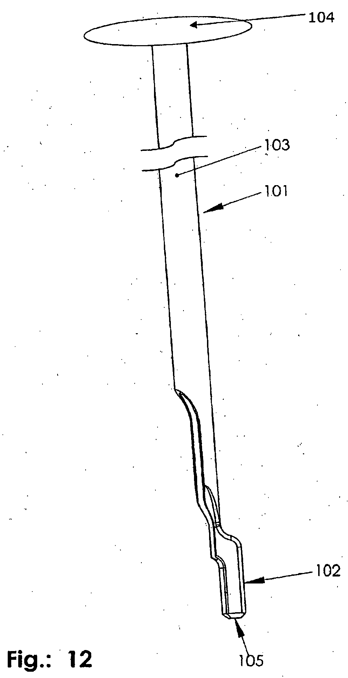

- Figur 12:: eine Seitenansicht des Gegenhalters,
- Figur 13:: eine perspektivische Ansicht des Betätigungsinstruments in Spreizstellung mit Betätigungshülse,
- Figur 14:: eine Draufsicht auf die Positionierscheibe von unten,
- Figur 15:: eine Seitenansicht des Betätigungsinstruments in Spreizstellung ohne Gehäuse,
- Figur 16:: eine Gesamtansicht des Betätigungsinstruments in Ruhestellung,
- Figur 17:: das Implantat mit Betätigungsinstrument in Ruhestellung,
- Figur 18:: das Implantat mit Betätigungsinstrument in Spreizstellung.

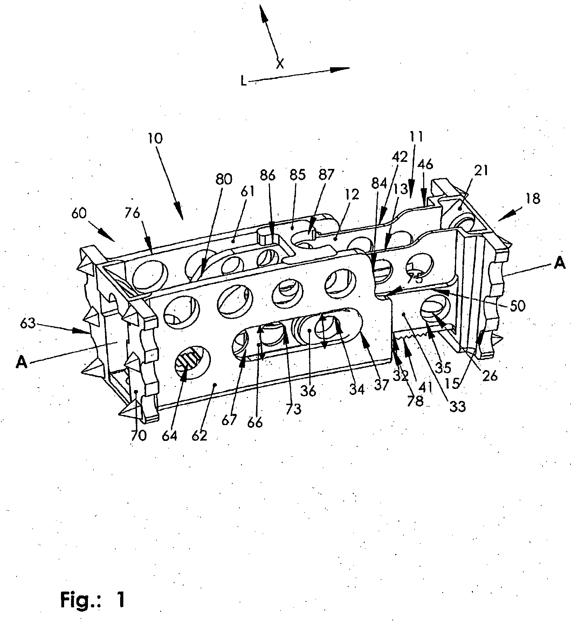

Das in Figur 1 dargestellte Implantat umfasst einen inneren Korb 11 und einen äußeren Korb 60, die Körbe 11, 60 sind U-förmig und der innere Korb 11 wird von dem äußeren Korb 60 umgriffen und ist in diesem Korb 60 teleskopartig geführt. Beide Körbe 11, 60 sind gleich ausgerichtet, d. h. die Schenkel 61, 62, 12, 13 der beiden Körbe und auch die Böden 16, 65 der beiden Körbe 11, 60 grenzen in der Ausgangsposition aneinander an, d. h., das Implantat ist ebenfalls U-förmig.

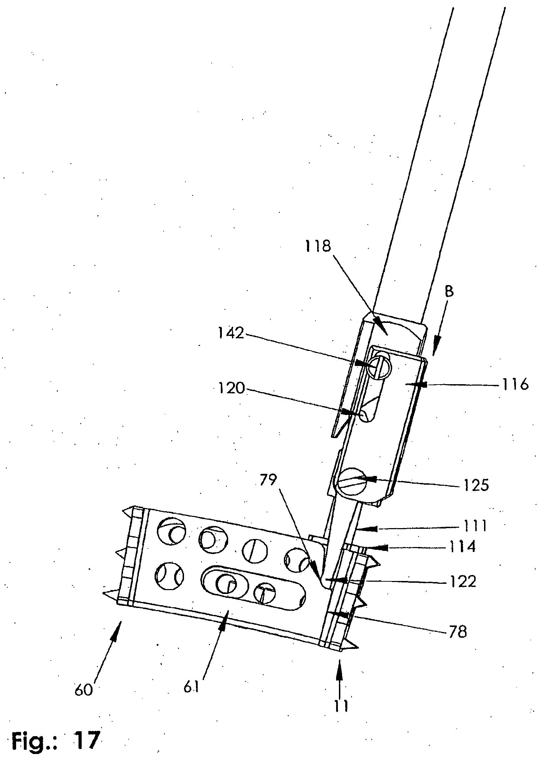

In der Ausgangs- bzw. Ruheposition, d. h. der Innenkorb 11 ist in den Außenkorb 60 vollständig hineingeschoben (vgl. auch Fig. 17), liegt die Seitenkante 78 der Schenkel 61, 62 des Außenkorbs an der nach innen weisenden Seite 15 der Wirbelstützfläche 19 des Innenkorbs 11 an und der Außenkorb 60 umgreift den Innenkorb 11 bis auf dessen Stirnseite 18 vollständig.

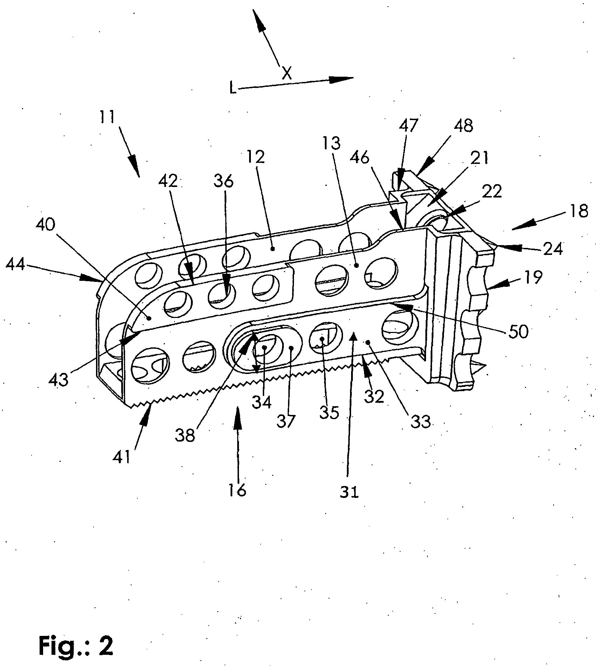

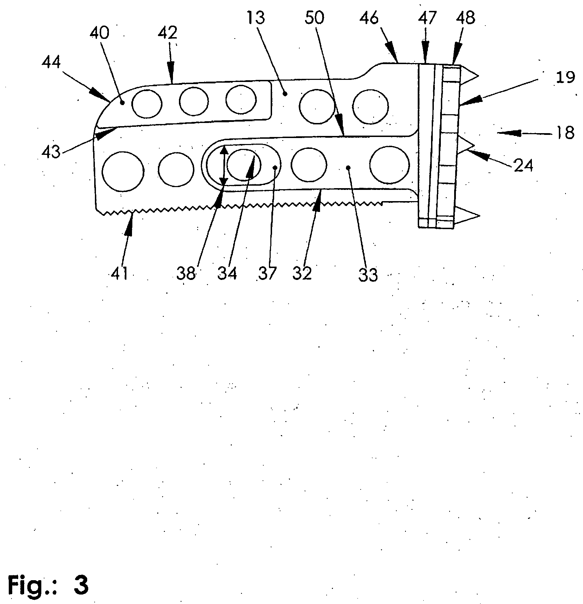

Der innere Korb 11 **(****Figur 2 - 5****)** weist zwei Schenkel 12, 13 mit kreisförmigen Durchbrüchen bzw. Ausnehmungen 34, 35, 36 auf. Die Durchbrüche 34, 35, 36 dienen zur besseren Durchknöcherung. Entsprechende Ausnehmurigen und Durchbrüche 64 befinden sich auch in den Schenkeln 61, 62 des Aüßenkorbs 60.

Das Implantat ist bezüglich der Ebene (xL-Ebene) in Richtung der Längsachse L spiegelsymmetrisch.

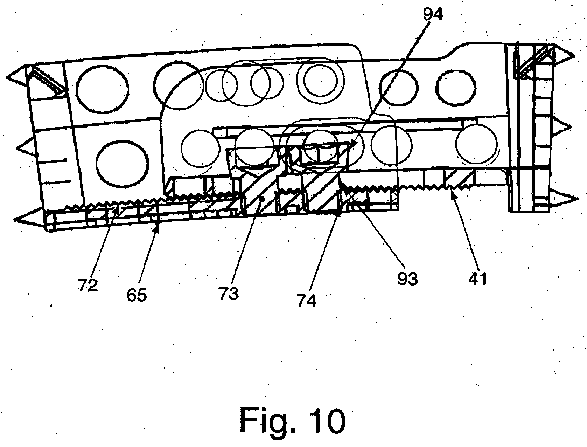

Die die beiden Schenkel 12, 13 verbindende Bodenseite 16 des Innenkorbs 11 weist ein Langloch 17 auf, das sich in Längsrichtung L des inneren Korbes 11 erstreckt. Die beiden Kanten 14 des Langlochs 17 verlaufen dabei parallel zueinander. Dieses Langloch 17 dient dem Feststellen oder dem Zwischenfixieren einer Einstellposition mittels einer Feststellschraube 73 **(****Fig. 1****,** **Fig. 10****).**

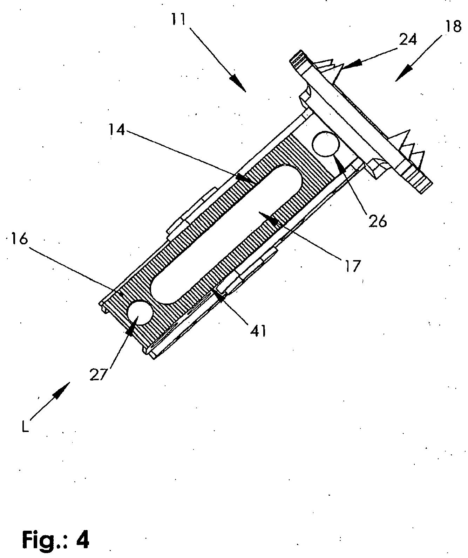

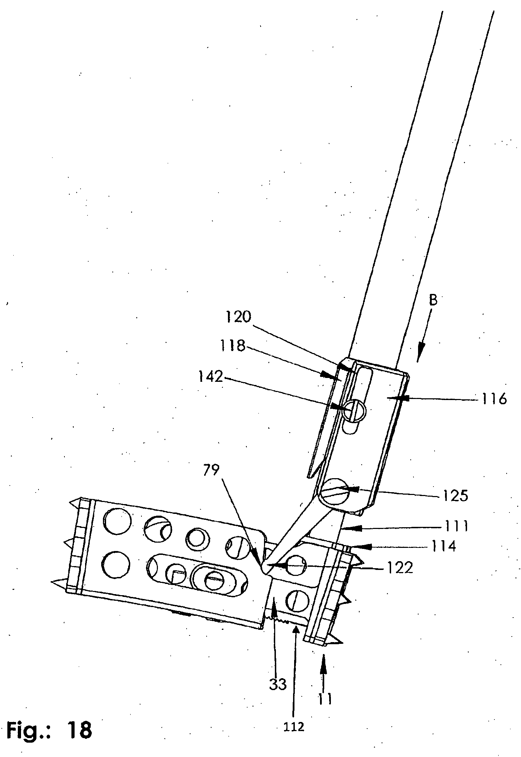

Zwischen dem Langloch 17 und der Stirnseite 18 des Innenkorbs 11 (vgl. **Figur 4****)** ist in der Bodenseite 16 des Innenkorbs 11 eine weitere Bohrung 26 vorgesehen, die dem Eingriff des Betätigungsinstruments 110 dient. Die Bohrung 26 umfasst ein Gewinde, so dass der Fixierungsstab 111 des Betätigungsinstruments 110 mit dem Außengewinde 112 in die Bohrung 26 eingeschraubt werden und damit das Betätigungsinstrument 110 mit dem Innenkorb 11 starr verbunden werden kann **(****Fig. 18****).**

Auf der dieser Bohrung 26 gegenüberliegenden Seite des Langlochs 17 ist in der Unterseite 16 des Innenkorbs 11 eine weitere Bohrung 27 vorgesehen.

Die Öffnungen bzw. Bohrungen 17, 27 im Boden 16 dienen zur optischen Kontrolle während bzw. nach der Implantateinbringung mit Blick in den Spinalkanal bzw. auf die Dura. Dies erhöht die Sicherheit der Implantation und mindert die Strahlenbelastung durch Verringerung der Zahl erforderlicher Röntgenkontrollen.

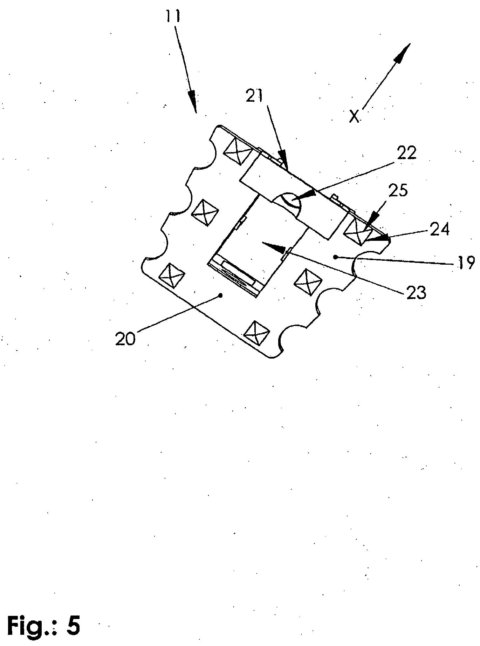

An der Stirnseite 18 des Innenkorbes 11 **(****Fig. 5****)** sind Wirbelstützflächen 19 oder Stege 20 sowie abwechselnd zu diesen Stützflächen 19 oder Stegen 20 sich in Längsrichtung L erstreckende zackenartige Arretierungsvorsprünge 24 vorgesehen. Die Wirbelstützflächen 19 oder Stege 20 sind gegenüber den Stirnflächen der Schenkel 12, 13 verbreitert, um eine hinreichend große Auflage für die Wirbelkörper bereitzustellen und eine feste Verankerung des Implantats an der Endplatte des Nachbarwirbels zu erlauben. An den Wirbelstützflächen 19 können abgeschrägte Endplatten 21 mit Bohrungen 22 vorgesehen sein, durch die der Innenkorb 11 falls gewünscht mit den Wirbelkörpern verschraubt werden kann.

Zwischen den Wirbelstützflächen 19 und dem Steg 20 befindet sich eine Öffnung 23 (Figur 5), die der besseren Durchknöcherung dient.

Die Arretierungsvorsprünge 24 an den jeweiligen Wirbelstützflächen 19 können an ihren Spitzen einen Schliff 25 zum oberflächlichen Eindringen und zur optimalen Verankerung an der jeweils gegenüberliegenden Endplatte des Nachbarwirbelkörpers umfassen.

Die Stirnseite 63 und Wirbelstützfläche 70 des Außenkorbes 60 ist entsprechend der Stirnseite 18 des inneren Korbes 11 ausgebildet.

Die Stirnseiten 18, 63 der Körbe 11, 60 verlaufen quer zur Längsrichtung L, d. h. der Verschieberichtung der Körbe 11, 60.

An den Außenseiten der Schenkel 12, 13 des inneren Korbs 11 ist jeweils ein vorspringender Abschnitt 33 vorgesehen, der die runden Öffnungen 34, 35 umfasst, die zum knöchernen Einwachsen dienen.

Die nach oben weisende Seite 50 des Abschnitts 33 bildet eine Führung für die beiden Hebelenden 122 des eingeschraubten Instruments 110. Bei Betätigung des Instruments 110 werden die Schiebeelemente bzw. Hebelenden 122 entlang der Kante 50 in L-Richtung geführt und durch die Bewegung der Hebelenden/Schiebeelemente 122 in L-Richtung des Außenkorbs 60 verschoben. Die Führungskante 50 weist ebenfalls die geringfügig von der Geraden abweichende Lordoseform auf.

Der Abschnitt 33 umfasst einen weiteren Vorsprung 37, dessen Außendurchmesser 38 in X-Richtung geririgfügig kleiner als der Innendurchmesser 66 des korrespondierenden Langlochs 67 in den Schenkeln 61, 62 des Außenkorbes 60 ist. Der Vorsprung 37 wird in dem Langloch 67 geführt und begrenzt die Verschiebbarkeit des inneren Korbs 11 gegenüber dem äußeren Korb 60 in L-Richtung und verhindert eine Verschiebung der Körbe 11, 60 gegeneinander in X-Richtung. In dem Führungsvorsprung 37, der eine annähernd ovale Form aufweisen kann, kann auch die Öffnung 34 vorgesehen sein.

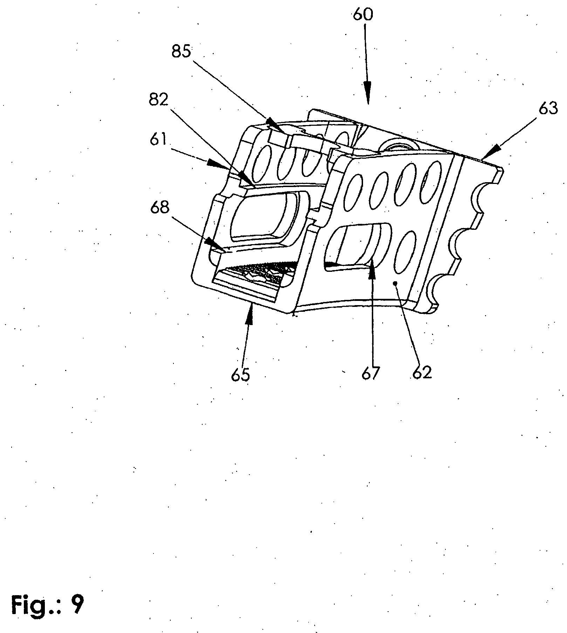

An der Außenseite der Schenkel 12, 13 des Innenkorbs 11 ist im oberen Bereich an dem der Stirnseite 18 gegenüberliegenden Ende ein weiterer Vorsprung 40 vorgesehen. Die Unterkante 43 des Vorsprungs 40 weist ebenfalls eine geringfügige, der Lordoseform folgende Krümmung auf und wird an einer korrespondierenden Kante 82 (Figur 9) an der Innenseite der Schenkel 61, 62 des Außenkorbes 60 geführt. Im Bereich der Vorsprünge 40 können ebenfalls kreisförmige Öffnungen 36 vorgesehen sein, die der Durchknöcherung dienen.

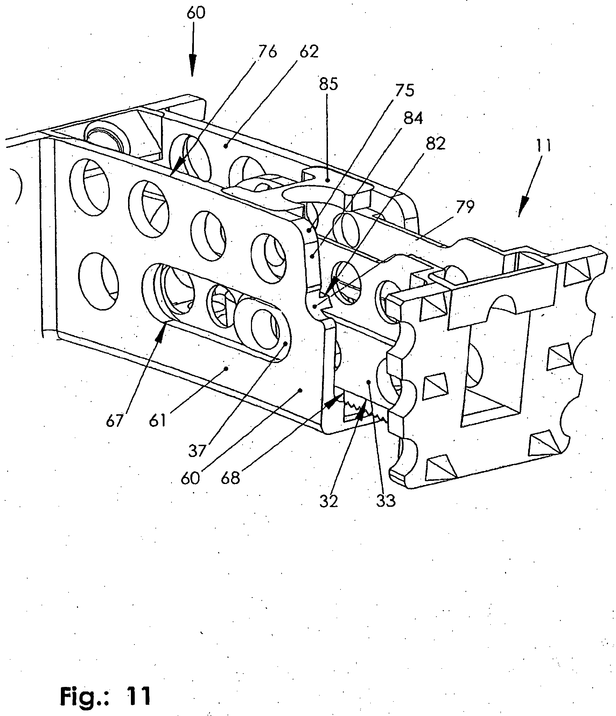

Im bodennahen Bereich der nach außen weisenden Seiten der Schenkel 12, 13 erstreckt sich in Längsrichtung die Unterkante 32 des Vorsprungs 33, die der Führung einer korrespondierenden Schiene 68 im nach innen weisenden bodennahen Eckbereich des Außenkorbs 60 dient **(****Figur 9****,** **Fig. 11****).**

Der ringförmige Vorsprung 37 und Langloch 67, Unterkante 43 des Vorsprungs 40 und Führungskante 82, sowie Kante 32 und Schiene 68 sind korrespondierende, am Außenkorb 60 und Innenkorb 11 vorgesehene Führungselemente, die alle in L-Richtung in einer geringfügig von einer Geraden abweichenden Form verlaufen. Sie dienen dazu, dass der innere Korb 11 gegenüber dem äußeren Korb 60 in einer der lordotischen Form, z.B. der Halswirbelsäule entsprechenden geringen Krümmungslinie ohne Verkantung verschoben werden kann.

Der Endbereich der Schenkel 12, 13 in L-Richtung geht an der der Stirnseite 18 des inneren Korbs 11 gegenüberliegenden Seite in Form eines Bogens 44 in die Oberseite 42 der Schenkel 12, 13 über, damit das Implantat vollständig schließbar ist.

An der Schenkeloberseite 42 nahe der Stirnseite 18 steigt die Schenkeloberseite 42 bis zum Erreichen der Höhe der Stirnseite 18 an, so dass die Oberseite 46 des Schenkels 12, 13 nahe der Stirnseite und die Oberseite 47 des Stirnbereichs eine durchgehende Auflagefläche für die Positionierscheibe 114 des Instruments 110 bilden.

An der Unterseite des Bodens 16 des Innenkorbs 11 ist im Bereich des Langlochs 17 eine Verzahnung 41 vorgesehen, die zur besseren Fixierung des inneren Korbs 11 gegenüber dem Außenkorb 60 durch zusätzliche Verrastung nach erfolgter Verschraubung dient.

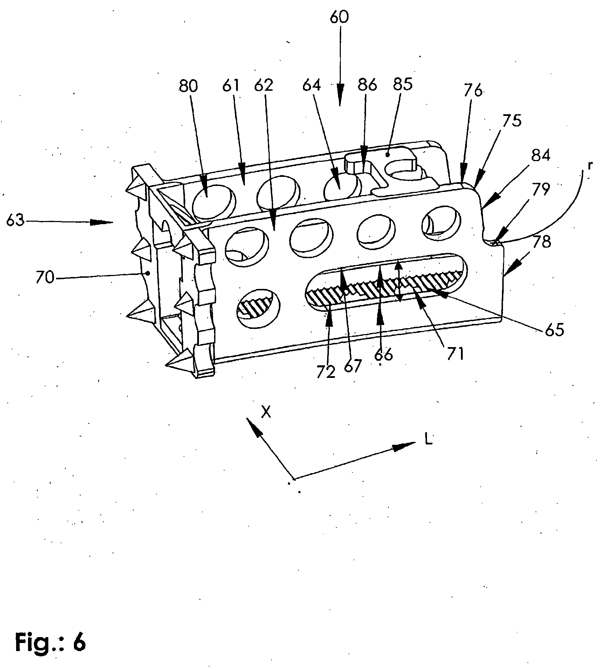

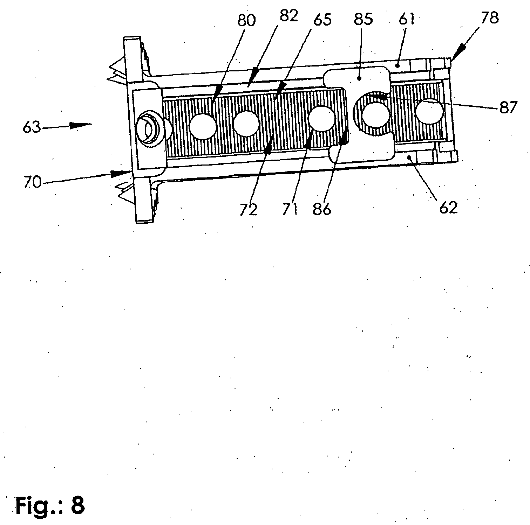

Der Außenkorb 60 **(****Figur 6****,** **7****,** **8****)** ist an der Stirnseite 63 entsprechend der Stirnseite 18 des Innenkorbs 11 ausgebildet und weist Wirbelstützflächen 70 auf, die den beim Innenkorb 11 beschriebenen Wirbelstützflächen 19 entsprechen.

Der Boden 65 des Außenkorbes 60 umfasst ebenfalls mehrere Bohrungen oder Durchbrüche 71, die der besseren Durchknöcherung dienen und der visuellen Kontrolle während der Implantation. An der Oberseite des Bodens 65 sind Verzahnungen 72 vorgesehen, die dem Eingriff in die korrespondierenden Verzahnungen 41 an der Außenseite des Bodens 16 des Innenkorbs 11 dienen.

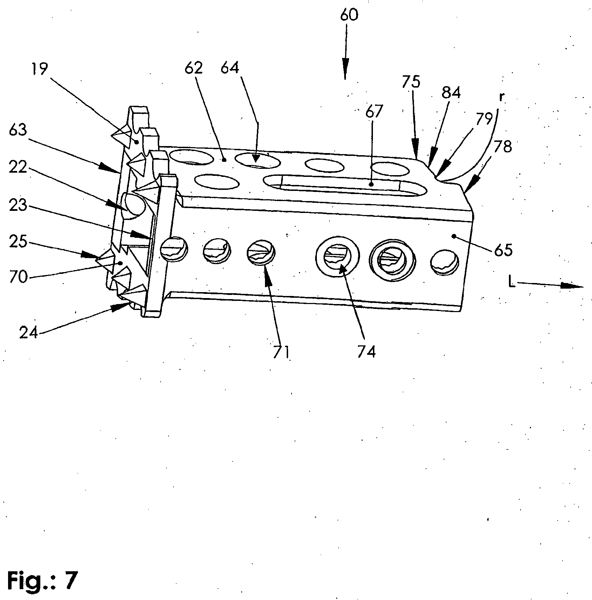

In dem Boden 65 des Außenkorbs 60 sind weiterhin Schrauben 73 in der Gewindebohrung 74 des Außenkorbs 60 vorgesehen **(****Figur 7****,** **10**), wobei der Schraubenschaft 93 durch das Langloch 17 im Boden des inneren Korbes 11 hindurchgeführt ist und der Schraubenkopf 94 auf der Oberseite des Bodens 16 des Innenkorbs 11 aufliegt. Der Innenkorb 11 ist bei gelockerter Schraube 73 gegenüber dem Außenkorb 60 verschiebbar. Wird die Schraube 73 festgeschraubt, so greifen die Verzahnungen 41, 72 ineinander und die Spreizposition ist fixiert. Die Fixierschraube 142 ist gegen das Herausfallen gesichert.

Die Länge des Schraubenschaftes 93 und die Größe der Bohrung 74 sind dabei so dimensioniert, dass auch bei festsitzender Schraube 73 in der Bohrung 74 das Schraubenende nicht über die Unterseite des Bodens 65 des Außenkorbs 60 hinaussteht.

Die Unterseite des Bodens 65 des Außenkorbes 60 ist bis auf die leicht lordotische Krümmung eben und weist die aus dem Stand der Technik bekannte Wandverstärkung, durch die es zu Komplikationen kommen kann, nicht auf.

Je nach Größe des Implantats kann die Anzahl an Schrauben 73 und Bohrungen 74 auch auf zwei oder mehr erhöht werden.

Die beiden Schenkel 61, 62 des Außenkorbs 60 weisen ebenfalls Öffnungen 64 auf, die der besseren Durchknöcherung dienen und das bereits zuvor beschriebene Langloch 67, das zusammen mit dem Vorsprung 37 im inneren Korb 11 der Führung dient. Die beiden zueinander parallelen Längsseiten des Langlochs 67 verlaufen dabei nicht vollständig gerade, sondern etwas gekrümmt, der Lordoseform folgend.

Die Seitenkante 78 der Schenkel 61, 62 des Außenkorbs 60 geht in L-Richtung auf der der Stirnseite 63 gegenüberliegenden Seite in Form eines Bogens 75 in die Oberseite 76 der Schenkel 61, 62 über. Die Seitenkante 78 der Schenkel 61, 62 besitzt eine Stufe 79, die in einem Kreisbogen mit dem Radius r in den leicht abgeschrägten oberen Bereich 84 der Seitenkante 78 übergeht.

Die Stufe 79 dient zum Abstützen der Hebelenden/Schiebeelemente 122 des Betätigungsinstruments 110.

An der Oberseite 76 der Schenkel 61, 62 ist zudem ein Steg 85 vorgesehen, der die offene Seite 80 des Implantats überbrückt und der mit beiden Schenkeln 61, 62 verbunden ist.

Der Steg 85 dient zur Anlage der Instrumente wie einem Schraubendreher oder dem nachfolgend beschriebenen Gegenhalter 101.

Hierzu weist der Steg 85 beidseitig Ausnehmungen 86, 87 auf, die der Aufnahme dieser Instrumente dienen. Die U-förmige Ausnehmung 86 dient zur Positionierung eines entsprechend geformten Gegenhalters 101 (Rotationshalter), der beim Einschrauben der Schrauben zur Aufnahme des Drehmoments dient und so eine Übertragung des Drehmoments auf das Implantat verhindert.

Der Gegenhalter 101 (**Fig**. **12****)** umfasst eine Stange 103 mit Griff 104 und im unteren Bereich eine zu der Ausnehmung 86 in dem Steg 85 korrespondierende rechteckige Stangenform, so dass der Gegenhalter 101 mit seiner Außenwand 102 im Endbereich an den Außenflächen der U-förmigen Ausnehmung 86 im Steg 85 anliegen kann.

Die kreisförmige Ausnehmung 87 im Steg 85 dient entsprechend als Führung für einen Schraubendreher oder Inbusschlüssel. Die Oberseite 42 der Schenkel 12, 13 des Innenkorbs 11 verläuft unterhalb des Überbrückungsstegs 85, die Unterseite des Überbrückungsstegs 85 bildet eine weitere Führung.

Außenkorb 60 und Innenkorb 11 sind über die offene Seite 80 (d. h. aus +-Richtung) mit Knochenspänen oder Knochenersatzmaterial befüllbar. Eine knöcherne Durchbauung mit den Nachbarwirbeln ist durch die Öffnung 23 In den Wirbelstützflächen 19, 70 und die Öffnungen 34, 35, 36, 64, 71, 67 möglich.

Die offene Seite 80 liegt den Böden 16, 65 der beiden Körbe 11, 60 gegenüber.

Außenkorb 60 und Innenkorb 11 bilden eine unverlierbare Einheit.

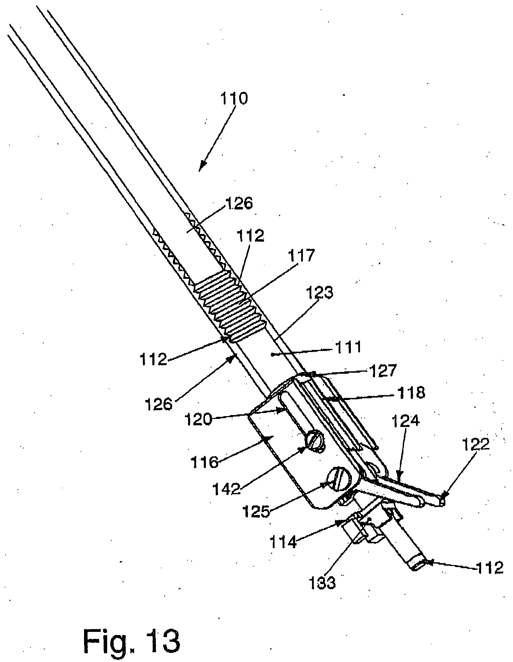

Zum Aufspreizen der beiden Körbe 11, 60 dient ein Betätigungsinstrument 110 **(****Figur 13****),** das den starren Fixierungsstab 111 und den Spreizmechanismus umfasst, der eine Schwenkbewegung der Hebelenden 124, die die Schiebeelemente 122 bilden, bewirkt. Am unteren Ende des Fixierungsstabes 111 ist ein Außengewinde 112 vorgesehen, das durch eine Drehbewegung am Fixierungsstab 111 in das Gewinde der Bohrung 26 in den Boden 16 des Innenkorbs 11 eingeschraubt werden kann.

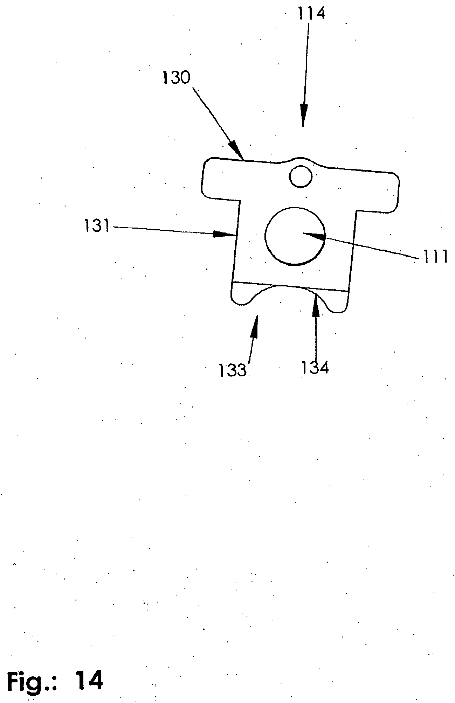

Oberhalb des Gewindes 112 ist - etwa in einer Höhe, die der Höhe des Innenkorbs 11 entspricht - am Fixierungsstab 111 eine Positionierscheibe 114 **(Fig. 13, 14)** vorgesehen, deren Kontur etwa der Draufsicht auf die offene Seite 80 im stirnseitigen Bereich des Innenkorbes 11, die ebenfalls etwa T-förmig ausgebildet ist, entspricht. Wird der Fixierungsstab 111 von oben in die offene Seite 80 des Innenkorbes 11 eingeführt und festgeschraubt, so liegt die Positionierscheibe 114 mit ihrer Unterseite auf den Oberseiten 46 der Schenkel 12, 13 und der Oberseite 47 des Stirnbereichs auf. An der einen Seite 133 der annähernd T-förmigen Positionierscheibe 114 **(Fig. 13, 14)** ist wieder eine halbkreisförmige Ausnehmung 134 zur Aufnahme des Fixierungsstabs 111 vorgesehen.

Die Positionierscheibe 114 ist verdrehbar, aber unverschiebbar an dem Fixierungsstab 111 befestigt. Die Hebelenden 122 des Instruments 110 werden entlang der Seitenkanten 131 der T-förmigen Positionierscheibe 114 geführt.

Auf diese Weise sind Positionierscheibe 114, Hebelenden/Schiebeelement 122 und der gesamte mit den Hebelenden/Schiebeelement 122 verbundene Spreizmechanismus zueinander positioniert und ermöglichen die Drehbewegung des Fixierungsstabs 111 gegenüber diesen den Fixierungsstab 111 umgebenden Elementen, dem Spreizmechanismus und der mit dem Spreizmechanismus über den Fixierungsstab 111 und die Bohrung 140 verbundenen Positionierscheibe 114.

Oberhalb der Positionierscheibe 114 ist der Spreizmechanismus vorgesehen, der auf dem Fixierungsstab 111 verschiebbar ist. Der Verschiebeweg ist durch die Positionierscheibe 114 und die Gewindespindel 117 begrenzt.

Der Spreizmechanismus umfasst ein Kraftübertragungselement 118, das im unteren seitlichen Bereich eine schräge Druckfläche 119 aufweist. Weiterhin umfasst der Spreizmechanismus einen das Kraftübertragungselement 118 durchquerenden Führungsstift 142, der in einem parallel dem Fixierungsstab 111 verlaufenden Langloch 120 zwangsgeführt ist. Das Langlochs 120 befindet sich in einem das Kraftübertragungselement 118 umgebenden U-förmigen Gehäuse 116. Am unteren Ende des Gehäuses 116 sind die Hebelelemente 124 vorgesehen, die drehbar um die Achse 125 gelagert sind. Die oberen Enden 128 der Hebelelemente 124 sind angeschrägt.

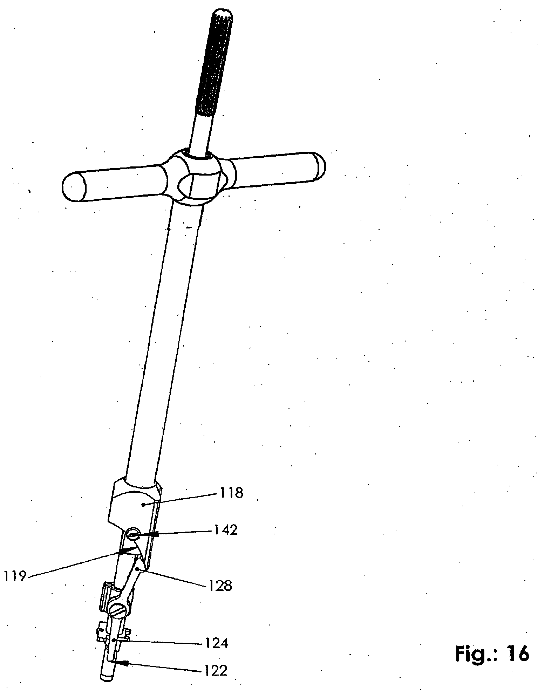

In Ruheposition wirkt keine Kraft auf das Kraftübertragungselement 118, der Führungsstift 142 befindet sich am oberen Ende des Langlochs 120 und das obere Ende 128 der Hebelelemente 124 liegt an der Schräge 119 in Ruheposition **(Fig. 16, 17).** Hebelelemente 124 und Hebelenden/Schiebeelemente 122 verlaufen im Wesentlichen parallel dem Fixierungsstab 111 nach unten. Hierzu ist ein Federelement vorgesehen, das die Hebelenden 122 in Ruheposition hält.

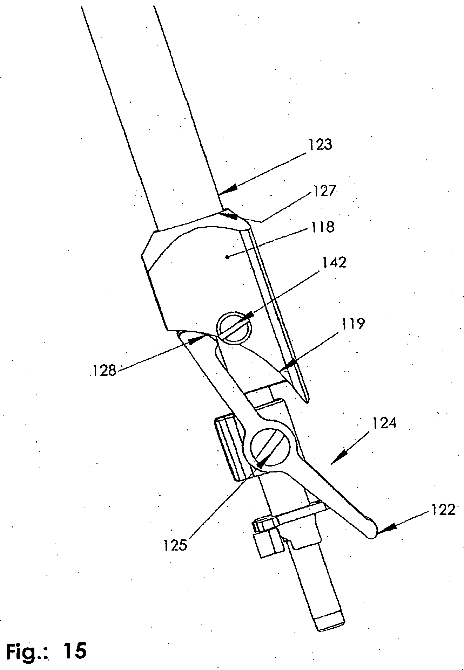

Wird nun das Kraftübertragungselement 118 entlang des Fixierungsstabes 111 nach unten geschoben **(****Fig. 15**, **Fig. 18** (Pfeil B)), so drückt die schräge Druckfläche 119 auf das obere Hebelende 128, so dass das untere Ende 122 des Hebelelements 124 gegenüber der Ruheposition nach außen geschwenkt wird.

Bei dieser Verschiebebewegung des Kraftübertragungselements 118 ist dieses in dem Langloch 120 in dem U-förmigen Gehäuse 116 zwangsgeführt. Durch diese Verschiebebewegung bewegen sich die Hebelenden/Schiebeelemente 122 auf einer Kreisbahn.

Nachdem die Hebelenden/Schiebeelemente 122 in Ruheposition in der Stufe 79 an der Seitenkante 78 des äußeren Korbs 60 anliegen **(****Fig. 17****),** führt die Schwenkbewegung des Hebelendes 122 des Instruments 110 auf der Kreisbahn und die Führung entlang der oberen Kante 50 des Abschnitts 33 im inneren Korb 11 zu einer Verschiebung des äußeren Korbs.

Eine fein abgestimmte Verschiebebewegung des Kraftübertragungselements 118 entlang des mit dem Innenkorb 11 verschraubten Fixierungsstabs 111 kann durch eine hülsenförmiges Betätigungselement 123, das nahe dem unteren Ende ein Innengewinde 126 aufweist, das mit der Gewindespindel 117 an der Fixierungsstab 111 korrespondiert, erreicht werden.

Durch das dosierte Einschrauben des Innengewindes 126 auf die Gewindespindel 117 bewegt sich die Unterkante 127 der Betätigungshülse 123 nach und nach nach unten und drückt durch diese Bewegung auf das Kraftübertragungselement 118, durch dessen Verschiebung die Hebelenden 122 ausgelenkt werden und damit der Außenkorb gegenüber dem Innenkorb 11, der mit der Fixierungsstab 111 starr verbunden ist, verschoben wird.

Sämtliche am unteren Ende des Instruments 110 vorgesehene Elemente sind derart ausgebildet, dass die schlanke Form des Fixierungsstabes 111 im Wesentlichen erhalten bleibt und somit keine sperrigen Vorrichtungen vorgesehen sind, die das Operationsfeld verdecken.

Ein weiterer Vorteil des Instruments 110 ist, dass dieses nahe der Stirnseite 18 des Innenkorbs 11 im Innenkorb 11 befestigt wird und somit die Beeinträchtigung der Sicht auf den Operationsraum durch das Instrument 110 so gering wie möglich gehalten wird.

Infolge der schlanken Gestalt der Bedienteile, der raumsparenden Rotationsbewegung der Hülse 123 zur Betätigung des Spreizmechanismus, der Platzierung des Instruments in einer Weise, dass keine Berührung mit den Valven erfolgt, kann der gesamte Operationsschnitt kleiner als bei den bislang bekannten Corpektomie-Körben ausfallen.

Ein weiterer wesentlicher Vorteil der vorliegenden Erfindung ist, dass eine verbesserte Röntgenkontrolle während der Implantation erfolgen kann, da die Sicht durch das schlanke Instrument 110 nur geringfügig versperrt wird - ein wesentlicher Unterschied der bislang bekannten großen, zangenartigen Bedienelemente.

Das erfindungsgemäße Instrument 110 basiert auf einer Trennung der Funktionen "Fixieren" und "Spreizen". Die Fixierung des Instruments 110 bzw. des Fixierungsstabs 111 erfolgt zunächst durch Befestigung des Außengewindes 112 am Ende des Fixierungsstabs 111 in der Gewindebohrung 26. Erst wenn der Fixierungsstab 111 fest in die Gewindebohrung 26 eingeschraubt ist, wird das hülsenförmige Betätigungselement 123 auf die Gewindespindel 117 aufgeschraubt und dadurch der Schwenkmechanismus auf die Hebelenden 122 aktiviert.

Ein Herausrutschen des Fixierungsstabs 111 während der Verschiebung des Außenkorbs 60 gegenüber dem Innenkorb 11 wird verhindert, da die Drehbewegung auf die Betätigungshülse 123 von der Drehbewegung auf das Außengewinde 112 im Fixierungsstab 111 entkoppelt ist.

Durch ein "Abschrauben" der Betätigungshülse 123 wird das Kraftübertragungselement 118 wieder nach oben und die Hebelenden 122 wieder in Richtung Ruheposition geschwenkt, so dass mit diesem Implantat 10 und diesem Instrument 110 eine einfach bedienbare, sicher funktionierende Überspreizung der Körbe 11, 60 wieder rückgängig gemacht werden kann.

Als Schraubendreher wird vorzugsweise ein Inbusschlüssel eingesetzt, der die Sicht auf das Operationsfeld ebenfalls durch eine schlanke Form nahezu nicht behindert.

Die in dem Außenkorb 60 und Innenkorb 11 in den Schenkeln 12, 13, 61, 62 vorgesehenen Öffnungen ermöglichen auch die Kontrolle des Sitzes des Implantats, der durch seitliches Röntgen kontrolliert werden kann.

Infolge der lordosen Form des erfindungsgemäßen Corpektomie-Korbs ist zudem kein Zusatzimplantat mehr erforderlich.

Durch das erfindungsgemäße Instrument 110 und das erfindungsgemäße Implantat 10 wird eine der Lordoseform des Corpektomie-Korbs folgende Spreizbewegung ermöglicht, ohne dass es zu Verkantungen der beiden Körbe gegeneinander während des Spreizvorgangs kommt.

Die Bedienbarkeit kann zudem dadurch verbessert werden, dass Innenkorb 11 und Gehäuse 116 des Instruments 110 farblich markiert sind, so dass die richtige Orientierung des Instruments 110 gegenüber dem Implantat auch farblich unterstützt wird.

Der erfindungsgemäße Corpektomie-Korb zeichnet sich zudem dadurch aus, dass eine optimale Verknöcherung erzielt werden kann und dass bei Überspreizung die Position wieder auf einfache Weise rückgängig gemacht werden kann.

## Patentansprüche

1. Implantatvorrichtung umfassend ein Betätigungsinstrument (110) und ein höhenverstellbares Implantat (10) mit einem im Wesentlichen U-förmigen inneren Korb (11) und einem U-förmigen äußeren Korb (60), wobei der innere Korb (11) des Implantats (10) von dem äußeren Korb (60) umgriffen und teleskopartig geführt wird und innerer Korb (11) und äußerer Korb (60) gleich ausgerichtet sind und wobei die Schenkel (12, 13) des inneren Korbs (11) und die Schenkel (61, 62) des äußeren Korbs (60) so ausgerichtet sind, dass sich eine im Wesentlichen durchgehende offene Seite (80) ergibt, die den Böden (16, 65) der beiden Körbe (11, 60) gegenüberliegt, und der innere (11) und äußere (60) Korb in einer vorgegebenen Endlage gegeneinander fixierbar sind, dass sich die Stirnseiten (18) der Körbe (11,60) quer zur Längsrichtung (L), der Verschieberichtung der Körbe (11, 60), erstrecken und an den Stirnseiten (18, 63) der Körbe (11, 60) Wirbelstützflächen (19, 70) vorgesehen sind, **dadurch gekennzeichnet,**
**dass** das Betätigungsinstrument (110) einen Fixierungsstab (111) und einen Spreizmechanismus mit Schiebeelementen (122) aufweist und Innenkorb (11) und Fixierungsstab (111) korrespondierende Elemente (26, 112) aufweisen, mittels derer eine lösbare starre Verbindung zwischen Fixierungsstab (111) und Innenkorb (11) erreicht wird, und wobei durch Betätigung des Spreizmechanismus an dem Betätigungsinstrument (110) der Außenkorb (60) durch Angriff der Schiebeelemente (122) an den Schenkeln (61, 62) gegenüber dem Innenkorb (11) in Längsrichtung (L) verschiebbar ist.

2. Implantatvorrichtung nach Anspruch 1, **dadurch gekennzeichnet,**
**dass** das Element (26) eine Bohrung (26) mit Gewinde im Boden (16) des Innenkorbs (11) ist.

3. Implantatvorrichtung nach einem der vorangegangenen Ansprüche, **dadurch gekennzeichnet,**
**dass** das Element (112) ein Außengewinde (112) am Fixierungsstab (111) ist.

4. Implantatvorrichtung nach einem der vorangegangenen Ansprüche, **dadurch gekennzeichnet,**
**dass** auf den Außenseiten der Schenkel (12, 13) des Innenkorbs (11) eine sich in Längsrichtung L ersteckende Stützfläche (50) vorgesehen ist, an der sich die Schiebeelemente (122) während der Betätigung des Spreizmechanismus des Betätigungsinstruments (110) abstützen.

5. Implantatvorrichtung nach einem der vorangegangenen Ansprüche, **dadurch gekennzeichnet,**
**dass** auf der der Stirnseite (63) des Außenkorbs (60) gegenüberliegenden Seite (78) der Schenkel (12, 13) eine Stufe (79) vorgesehen ist, die der Abstützung der Enden der Schiebeelemente (122) des Betätigungsinstruments (110) dient.

6. Implantatvorrichtung nach einem der vorangegangenen Ansprüche, **dadurch gekennzeichnet,**
**dass** der Außenkorb (60) einen die offene Seite (80) überbrückenden Überbrückungssteg (85), der mit den Schenkeln (61, 62) fest verbunden ist und der Ausnehmungen (86, 87) zur Anlage von Instrumenten umfasst, aufweist.

7. Implantatvorrichtung nach einem der vorangegangenen Ansprüche, **dadurch gekennzeichnet,**
**dass** an den Schenkeln (12, 13, 61, 62) der Körbe (11, 60) korrespondierende Elemente (67, 37; 32, 68; 43, 82) vorgesehen sind, die der Führung der Spreizbewegung der beiden Körbe (11, 60) in einer der lordotischen Form folgenden Bewegung dienen, wobei vorzugsweise die Elemente (67, 37; 32, 68; 43, 82) korrespondierende Stützflächen (32, 68; 43, 82) oder Öffnungen (67) mit in den Öffnungen geführten Vorsprüngen (37) sind und wobei vorzugsweise die Stützflächen (32, 68; 43, 82) und/oder die Öffnungen (67) und/oder die Vorsprünge (37) entlang der Längsachse L in einer geringfügig von der Geraden abweichenden Form ausgebildet sind.

8. Implantatvorrichtung nach einem der vorangegangenen Ansprüche, **dadurch gekennzeichnet,**
**dass** der Innenkorb (11) im Boden (16) ein Langloch (17) und der Außenkorb (60) im Boden (65) Öffnungen (71) aufweist und eine Schraube (73) die Körbe (11, 60) durch die Öffnungen (17, 71) verbindet.

9. Implantatvorrichtung nach einem der vorangegangenen Ansprüche, **dadurch gekennzeichnet,**
**dass** die Schenkel (12, 13; 61, 62) Öffnungen (34, 35, 36, 64) und/oder die Böden (16, 65) Öffnungen und/oder die Wirbelstützflächen (19, 70) Öffnungen (23) aufweisen, die der Durchknöcherung dienen.

10. Implantatvorrichtung nach einem der vorangegangenen Ansprüche, **dadurch gekennzeichnet,**
**dass** das Außengewinde (112) des Fixierungsstabs (111) am Stab-ende vorgesehen ist.

11. Implantatvorrichtung nach einem der vorangegangenen Ansprüche, **dadurch gekennzeichnet,**
**dass** die Schiebeelemente (122) und der Spreizmechanismus nahe dem Ende des Fixierungsstabs (111) vorgesehen sind.

12. Implantatvorrichtung nach einem der vorangegangenen Ansprüche, **dadurch gekennzeichnet,**
**dass** der Spreizmechanismus ein Kraftübertragungselement (118) mit einer schrägen Druckfläche (119) umfasst, die Hebelelemente (124), die die Schiebeelemente (122) umfassen, schwenkbar um eine Achse (125) gelagert sind und die oberen Enden der Hebelelemente (124) im Ruhezustand an der Druckfläche (119) anliegen und bei Krafteinleitung durch den Druck auf die schräge Druckfläche (119) des Kraftübertragungselements (118) die Schiebeelemente (122) aus der Ruhelage heraus geschwenkt werden.

13. Implantatvorrichtung nach einem der vorangegangenen Ansprüche, **dadurch gekennzeichnet,**
**dass** der Spreizmechanismus gegenüber dem Fixierungsstab (111) verschiebbar ist.

14. Implantatvorrichtung nach Anspruch 12 oder 13,
**dadurch gekennzeichnet,**
**dass** das Kraftübertragungselement (118) von einem U-förmigen Gehäuse (116) mit einem Langloch (120) umgeben ist und ein das Kraftübertragungselement (118) durchquerender Führungsstift (142), der in dem Langloch (120) zwangsgeführt ist, vorgesehen ist, wobei vorzugsweise das untere Ende des Gehäuses (116) eine Achse (125) durchquert, an der die Hebelelemente (124) schwenkbar befestigt sind.

15. Implantatvorrichtung nach einem der vorangegangenen Ansprüche, **dadurch gekennzeichnet,**
**dass** der Fixierungsstab (111) eine Gewindespindel (117) aufweist, auf deren Außengewinde eine Betätigungshülse (123) aufgeschraubt werden kann, wobei durch die Schraubbewegung die Unterkante (127) der Betätigungshülse (123) das Kraftübertragungselement (118) in Richtung Stabende nach unten drückt und/oder
**dass** der Fixierungsstab (111) eine Positionierscheibe (114) aufweist, die nahe des Stabendes verdrehbar, jedoch unverschiebbar gelagert ist.

16. Implantatvorrichtung nach einem der vorangegangenen Ansprüche, **dadurch gekennzeichnet,**
**dass** der Verschiebeweg des Spreizmechanismus entlang des Fixierungsstabs (111) durch die Positionierscheibe (114) und die Gewindespindel (117) begrenzt ist.

17. Implantatvorrichtung nach Anspruch 15 oder 16,
**dadurch gekennzeichnet,**
**dass** der Spreizmechanismus zusammen mit der Positionierscheibe (114) gegenüber dem Fixierungsstab (111) drehbar ist oder dass der Spreizmechanismus gegenüber der Positionierscheibe (114) nicht drehbar ist.

18. Implantatvorrichtung nach einem der vorangegangenen Ansprüche, **dadurch gekennzeichnet,**
**dass** zusätzlich ein Gegenhalter (101) vorgesehen ist, der an der Ausnehmung (86) an dem Überbrückungssteg (85) positioniert werden kann.

## Claims

1. An implant device comprising an actuating instrument (110) and a height-adjustable implant (10) having a substantially U-shaped inner cage (11) and a U-shaped outer cage (60), wherein the inner cage (11) of the implant (10) is embraced by the outer cage (60) and telescopically guided, and the inner cage (11) and outer cage (60) are equally aligned, and wherein the limbs (12, 13) of the inner cage (11) and the limbs (61, 62) of the outer cage (60) are aligned such that a substantially continuous lateral opening (80) results that lies opposite the bottoms (16, 65) of the two cages (11, 60), and the inner (11) and outer (60) cages can be fixed with respect to each other in a predetermined end position that the front faces (18) of the cages (11, 60) extend transversely to the longitudinal direction (L), the direction of the movement of the cages (11, 60), and vertebral support surfaces (19, 70) are provided on the front faces (18, 63) of the cages (11, 60), **characterized in that** the actuating instrument (110) has a fixation rod (111) and an expansion mechanism with sliding elements (122), and the inner cage (11) and fixation rod (111) have corresponding elements (26, 112), by means of which a releasable, rigid connection between the fixation rod (111) and inner cage (11) is achieved, and wherein, by actuating the expansion mechanism on the actuating instrument (110), the outer cage (60) can be moved in the longitudinal direction (L) relative to the inner cage (11) by engagement of the sliding elements (122) on the limbs (61, 62).

2. The implant device as claimed in claim 1, **characterized in that** the element (26) is a bore (26) with thread in the bottom (16) of the inner cage (11).

3. The implant device as claimed in one of the preceding claims, **characterized in that** the element (112) is an outer thread (112) on the fixation rod (111).

4. The implant device as claimed in one of the preceding claims, **characterized in that**, on the outsides of the limbs (12, 13) of the inner cage (11), a support surface (50) is provided which extends in the longitudinal direction L and on which the sliding elements (122) are supported during the actuation of the expansion mechanism of the actuating instrument (110).

5. The implant device as claimed in one of the preceding claims, **characterized in that**, on the side (78) of the limbs (12, 13) that lies opposite the front face (63) of the outer cage (60), a step (79) is provided which serves to support the ends of the sliding elements (122) of the actuating instrument (110).

6. The implant device as claimed in one of the preceding claims, **characterized in that** the outer cage (60) has a bridging web (85) which bridges the lateral opening (80) and which is rigidly connected to the limbs (61, 62) and comprises recesses (86, 87) for receiving instruments.

7. The implant device as claimed in one of the preceding claims, **characterized in that** corresponding elements (67, 37; 32, 68; 43, 82) are provided on the limbs (12, 13, 61, 62) of the cages (11, 60) and serve to guide the expansion movement of the two cages (11, 60) in a movement that follows the lordotic shape, wherein the elements (67, 37; 32, 68; 43, 82) are preferably corresponding support surfaces (32, 68; 43, 82) or openings (67) with projections (37) guided into the openings and wherein preferably the support surfaces (32, 68; 43, 82) and/or the openings (67) and/or the projections (37) along the longitudinal axis L are in a form slightly deviating from the straight.

8. The implant device as claimed in one of the preceding claims, **characterized in that** the inner cage (11) has an oblong hole (17) in the bottom (16) and the outer cage (60) has openings (71) in the bottom (65), and a screw (73) connects the cages (11, 60) through the openings (17, 71).

9. The implant device as claimed in one of the preceding claims, **characterized in that** the limbs (12, 13; 61, 62) have openings (34, 35, 36, 64) and/or the bottoms (16, 65) have openings and/or the vertebral support surfaces (19, 70) have openings (23), which allow bone to grow through.

10. The implant device as claimed in one of the preceding claims, **characterized in that** the outer thread (112) of the fixation rod (111) is provided on the rod end.

11. The implant device as claimed in one of the preceding claims, **characterized in that** the sliding elements (122) and the expansion mechanism are provided near the end of the rod (111).

12. The implant device as claimed in one of the preceding claims, **characterized in that** the expansion mechanism comprises a force transmission element (118) with an oblique pressing surface (119), the lever elements (124), which comprise the sliding elements (122), are mounted pivotably about an axis (125), and the upper ends of the lever elements (124) bear on the pressing surface (119) in the rest state, and, when force is introduced by the pressure on the oblique pressing surface (119) of the force transmission element (118), the sliding elements (122) are pivoted out of the rest position.

13. The implant device as claimed in one of the preceding claims, **characterized in that** the expansion mechanism is displaceable relative to the fixation rod (111).

14. The implant device as claimed in claim 12 or 13, **characterized in that** the force transmission element (118) is surrounded by a U-shaped housing (116) with an oblong hole (120), and a guide pin (142) is provided that passes through the force transmission element (118) and is forcibly guided in the oblong hole (120), wherein preferably the lower end of the housing (116) passes through an axis (125) on which the lever elements (124) are secured pivotably.

15. The implant device as claimed in one of the preceding claims, **characterized in that** the fixation rod (111) has a threaded spindle (117), onto the outer thread of which an actuating sleeve (123) can be screwed, wherein, by the screwing movement, the lower edge (127) of the actuating sleeve (123) presses the force transmission means (118) downward in the direction of the rod end and/or that the fixation rod (111) has a fixation disk (114) which is mounted so as to be rotatable near the rod end but not displaceable.

16. The implant device as claimed in one of the preceding claims, **characterized in that** the displacement path of the expansion mechanism along the fixation rod (111) is limited by the fixation disk (114) and the threaded spindle (117).

17. The implant device as claimed in claim 15 or 16, **characterized in that** the expansion mechanism, together with the fixation disk (114), is rotatable relative to the fixation rod (111) or that the expansion mechanism is not rotatable relative to the fixation disk (114).

18. The implant device as claimed in one of the preceding claims, **characterized in that** a retainer (101) is also provided, which can be positioned on the recess (86) on the bridging web (85).

## Revendications

1. Dispositif implantable comprenant un instrument d'actionnement (110) et un implant (10) réglable en hauteur avec une cage intérieure (11) essentiellement en forme de U et une cage extérieure (60) en forme de U, la cage intérieure (11) de l'implant (10) étant entourée par la cage extérieure (60) et guidée de manière télescopique, et la cage intérieure (11) et la cage extérieure (60) étant orientées de la même manière, et les branches (12, 13) de la cage intérieure (11) et les branches (61, 62) de la cage extérieure (60) étant orientées de manière à former un côté ouvert (80) essentiellement continu, lequel est situé à l'opposé des fonds (16, 65) des deux cages (11, 60) et les cages intérieure (11) et extérieure (60) pouvant être fixées l'une par rapport à l'autre dans une position finale prédéfinie de sorte que les faces frontales (18) des cages (11, 60) s'étendent perpendiculairement au sens longitudinal (L), sens de déplacement des cages (11, 60), et des surfaces d'appui de vertèbre (19, 70) étant prévues sur les faces frontales (18, 63) des cages (11, 60), **caractérisé en ce que**
l'instrument d'actionnement (110) présente une tige de fixation (111) et un mécanisme d'écartement avec des éléments coulissants (122) et **en ce que** la cage intérieure (11) et la tige de fixation (111) présentent des éléments correspondants (26, 112) au moyen desquels il est possible d'obtenir une liaison rigide amovible entre la tige de fixation (111) et la cage intérieure (11) et **en ce que** la cage extérieure (60) peut être déplacée en sens longitudinal (L) par rapport à la cage intérieure (11) par actionnement du mécanisme d'écartement situé sur l'instrument d'actionnement (110), et ce grâce à l'action des éléments coulissants (122) sur les branches (61, 62).

2. Dispositif implantable selon la revendication 1, **caractérisé en ce que** l'élément (26) est un trou (26) avec filetage ménagé dans le fond (16) de la cage intérieure (11).

3. Dispositif implantable selon l'une quelconque des revendications précédentes, **caractérisé en ce que**
l'élément (112) est un filet extérieur (112) situé sur la tige de fixation (111).

4. Dispositif implantable selon l'une quelconque des revendications précédentes, **caractérisé en ce que**
sur les faces extérieures des branches (12, 13) de la cage intérieure (11) est prévue une surface d'appui (50) qui s'étend en sens longitudinal L et contre laquelle s'appuient les éléments coulissants (122) pendant l'actionnement du mécanisme d'écartement de l'élément d'actionnement (110).

5. Dispositif implantable selon l'une quelconque des revendications précédentes, **caractérisé en ce que**
sur la face (78) des branches (12, 13) située à l'opposé de la face frontale (63) de la cage extérieure (60) est prévu un palier (79) qui sert d'appui aux extrémités des éléments coulissants (122) de l'élément d'actionnement (110).

6. Dispositif implantable selon l'une quelconque des revendications précédentes, **caractérisé en ce que**
la cage extérieure (60) présente une partie de rattachement (85) qui enjambe le côté ouvert (80) et est reliée de manière fixe aux branches (61, 62) et qui comprend des évidements (86, 87) servant d'appui à des instruments.

7. Dispositif implantable selon l'une quelconque des revendications précédentes, **caractérisé en ce que**
sur les branches (12, 13, 61, 62) des cages (11, 60) sont prévus des éléments correspondants (67, 37; 32, 68; 43, 82) qui servent au guidage du mouvement d'écartement des deux cages (11, 60) selon un mouvement suivant la forme lordotique, les éléments (67, 37; 32, 68; 43, 82) étant de préférence des surfaces d'appui (32, 68; 43, 82) correspondantes ou des ouvertures (67) correspondant avec des saillies (37) guidées dans lesdites ouvertures, et les surfaces d'appui (32, 68; 43, 82) et/ou les ouvertures (67) et/ou les saillies (37) étant réalisées de préférence le long de l'axe longitudinal L sous une forme qui diffère légèrement de la ligne droite.

8. Dispositif implantable selon l'une quelconque des revendications précédentes, **caractérisé en ce que**
la cage intérieure (11) présente dans le fond (16) un trou oblong (17) et que la cage extérieure (60) présente dans le fond (65) des ouvertures (71) et qu'une vis (73) relie les cages (11, 60) par les ouvertures (17, 71).

9. Dispositif implantable selon l'une quelconque des revendications précédentes, **caractérisé en ce que**
les branches (12, 13; 61, 62) présentent des ouvertures (34, 35, 36, 64) et/ou les fonds (16, 65) présentent des ouvertures et/ou les surfaces d'appui de vertèbre (19, 70) présentent des ouvertures (23) qui servent au passage du tissu osseux pendant sa croissance.

10. Dispositif implantable selon l'une quelconque des revendications précédentes, **caractérisé en ce que**
le filet extérieur (112) de la tige de fixation (111) est prévu à l'extrémité de la tige.

11. Dispositif implantable selon l'une quelconque des revendications précédentes, **caractérisé en ce que**
les éléments coulissants (122) et le mécanisme d'écartement sont prévus près de l'extrémité de la tige de fixation (111).

12. Dispositif implantable selon l'une quelconque des revendications précédentes, **caractérisé en ce que**
le mécanisme d'écartement comprend un élément de transmission de force (118) avec une surface de pression inclinée (119), **en ce que** les éléments de levage (124) qui comprennent les éléments coulissants (122) sont logés de manière à pivoter sur un axe (125) et que les extrémités supérieures des éléments de levage (124) reposent à l'état de repos contre la surface de pression (119) et **en ce que**, lors de l'application d'une force, les éléments coulissants (122) quittent la position de repos en effectuant un mouvement de pivotement généré par la pression exercée sur la surface de pression inclinée (119) de l'élément de transmission de force (118).

13. Dispositif implantable selon l'une quelconque des revendications précédentes, **caractérisé en ce que**
le mécanisme d'écartement peut coulisser par rapport à la tige de fixation (111).

14. Dispositif implantable selon la revendication 12 ou 13, **caractérisé en ce que**
l'élément de transmission de force (118) est entouré d'un boîtier (116) en forme de U avec un trou oblong (120) et **en ce qu'**il est prévu une tige de guidage (142) qui traverse l'élément de transmission de force (118) et qui est guidée de manière forcée dans le trou oblong (120), l'extrémité inférieure du boîtier (116) traversant de préférence un axe (125) sur lequel sont fixés les éléments de levage (124) de manière à pouvoir pivoter.

15. Dispositif implantable selon l'une quelconque des revendications précédentes, **caractérisé en ce que**
la tige de fixation (111) présente une broche filetée (117) sur le filet extérieur de laquelle peut être vissée une douille d'actionnement (123), le bord inférieur (127) de la douille d'actionnement (123) appuyant, par le mouvement de vissage, l'élément de transmission de force (118) vers le bas en direction de l'extrémité de la tige et/ou **en ce que**
la tige de fixation (111) présente un disque de positionnement (114) qui est logé près de l'extrémité de la tige de manière à pouvoir tourner sans pour autant pouvoir coulisser.

16. Dispositif implantable selon l'une quelconque des revendications précédentes, **caractérisé en ce que**
la distance de déplacement du mécanisme d'écartement est limitée le long de la tige de fixation (111) par le disque de positionnement (114) et la broche filetée (117).

17. Dispositif implantable selon la revendication 15 ou 16, **caractérisé en ce que**
le mécanisme d'écartement peut tourner ensemble avec le disque de positionnement (114) par rapport à la tige de fixation (111) ou **en ce que** le mécanisme d'écartement ne peut pas tourner par rapport au disque de positionnement (114).

18. Dispositif implantable selon l'une quelconque des revendications précédentes, **caractérisé en ce que**
il est prévu en outre un bras support (101) qui peut être positionné sur l'évidement (86) ménagé dans la partie de rattachement (85).
